# EUROPEAN PATENT SPECIFICATION

(11) **EP 1 495 729 B1**
(45) Date of publication and mention of the grant of the patent: **29.11.2006**
(21) Application number: 03015390.2
(22) Date of filing: 08.07.2003
(51) Int. Cl.: A61B 17/70, A61B 17/74, A61B 17/72, A61B 17/58, A61F 2/46

(54) **Flexible, expandable and air-permeable implant for medical application**
Flexibles, expandierbares und luftdurchlässiges Implantat für medizinische Zwecke
Dispositif flexible, expansible et perméable à l'air pour implantation dans le corps

(43) Date of publication of application: 12.01.2005
(73) Proprietor: Lin, Kwan-Ku, Taipei (TW)
(72) Inventor: Lin, Kwan-Ku, Taipei (TW)
(74) Representative: Grünecker, Kinkeldey, Stockmair & Schwanhäusser Anwaltssozietät

(56) References cited:
- EP-A- 1 132 051
- US-A- 3 980 177
- US-A- 6 017 366
- US-A1- 2002 068 974
- US-B1- 6 402 784

## Description

### FIELD OF THE INVENTION

The present invention relates generally to a medicinal filler, and more particularly to a compressible and breathable medicinal filler.

### BACKGROUND OF THE INVENTION

The conventional medical blockage of cavity often involves the use of a solid material, such as gypsum. Similarly, the fixation of an incision involves the use of a solid material. Such solid fillers as described above are used to bring about a fixation effect. However, the conventional medical fillers often fail to join together intimately, thereby resulting in formation of gaps among them. In another words, they are bound to loosen up easily due to poor frictional contact. The conventional cavity blockage is defective in design in that the filler and the cavity wall lack an intimate contact. In the case of the incision fixation, the filler is not properly shaped to fit well into the incision.

As related prior art US 2002/0068974 can be mentioned, which discloses the use of an expandable surgical fabric bag in a prepared cavity in bone in order to correct bone abnormalities. The bag is inflated using bone replacement material US-A-6,402,784 discloses an intervertebral disc nucleus prothesis shaped in the form of a cover and comprising a permeable layer of an immunologically neutral material and a woven or porous fabric layer into which a hydrogel can be introduced to counteract the high pressures encountered.

### SUMMARY OF THE INVENTION

The primary objective of the present invention is to provide a medicine filler, which is free of the drawbacks of the conventional medicinal fillers described above. The filler comprises a pasty medicinal material and a filling member which is flexible and breathable. The filling member is properly compressed before it is injected with the pasty medicinal material. Upon having been injected, the filling member returns to its original form.

It is another objective of the present invention to provide a medicine filling system comprising said pasty medicinal material, said filling member filler, and an injection tool by which the pasty medicinal material is put into the filling member.

Said flexible and breathable filling member is formed of one or more meshed walls and provided with a hollow receiving portion and an inlet in communication with said receiving portion, said meshed walls being elastically compressible and formed of laminated multi-layer walls, and each layer of which is provided with a plurality of meshes, so that said meshed walls are not air-tight and wherein the meshes of two adjacent layers of a wall are not aligned. Said pasty medicinal material is injected into said receiving portion via said inlet of said filling member.

Preferably, said each layer is provided with more than 6 meshes per linear inch (2.54 linear cm). Preferably, said each layer is provided with more than 20 meshes per linear inch. Preferably, said each layer is provided with more than 40 meshes per linear inch. Preferably, said each layer is provided with more than 100 meshes per linear inch.

Preferably, said filling member is shaped like a sac, bag, ball, rod, or cylinder.

Said injecting tool of the medical filler system of the present invention1 is detachably fastened with said inlet of said filling member, so that said pasty medicinal material is able to be injected into said receiving portion via said inlet of said filling member by said injecting tool.

Preferably, said injecting tool comprises a connection tube and an injecting cylinder, said connection tube being provided at one end with an outer threaded portion such that said outer threaded portion of said connection tube is engaged with an inner threaded portion of said inlet of said filling member; said injecting cylinder comprising a barrel and a plunger, said barrel being fastened with another end of said connection tube, wherein said barrel is used to hold said pasty medicinal material which is to be injected into said receiving portion of said filling member via said connection tube by a plunging motion of said plunger.

### BRIEF DESCRIPTION OF THE DRAWINGS

FIG. 1 shows a perspective view of a medicine filling system of the present invention.
FIG. 2 shows an exploded view of the medicine filling system of the present invention.
FIG. 3a shows a sectional schematic view of a meshed wall of a filling member not forming part of the present invention.
FIG. 3b shows a sectional schematic view of a laminated wall of the filling member of the present invention.
FIGS. 4a-4d are schematic view of the filling members of the present invention in various forms.
FIGS. 5a-5C are schematic views of the system of the present invention at work to bring about a blocking action in the surgical operation.

### DETAILED DESCRIPTION OF THE INVENTION

The present invention is characterized by the medicinal material which is injected in a pasty form, and by the filling member which is formed of one or more flexible and breathable walls. These walls are provided with a plurality of passages and are multi-layer walls made by laminating. The filling member is provided with a hollow receiving portion and an inlet via which the pasty medicinal material is injected into the receiving portion. Prior to injection of the pasty medicinal material into the filling member, the filling member is compressed. The injection action causes the filling member to return to its original form. The interior of the receiving portion of the filling member is in communication with the exterior of the filling member via the passages. As far as the passages are concerned, the filling member of the present invention is formed of the multi-layer walls, each layer having meshes in the range of 20-100 per linear inch (2,54 linear cm). If the filling member is formed of a 20-mesh wall, three or more layers laminated walls are suggested, depending on the viscosity of the pasty medicinal material and the content of grains in the medicinal material. For example, if the pasty medicinal material is poly(methyl methacrylate) (PMMA), the filling member is preferably formed of three layers of 20-mesh walls by laminating. This is due to the fact that PMMA has a relatively high viscosity and contains grains which are relatively large in size. If the pasty medicinal material has a relatively low viscosity and contains grains which are relatively small in size, the filling member is preferably formed of four or more layers of 20-mesh walls by laminating. The laminated structure of the filling member of the present invention is dependent on the viscosity of the pasty medicinal material or the particle size of grains contained therein, as exemplified by the experimental data listed in the following table.

| Experiment | Type of wall | No. of layers of walls | Form of filling member | Medicinal material | Leaking or not |
|---|---|---|---|---|---|
| 1 | 20-mesh wall | 3 | baglike, meshed | PMMA | No leak |
| 2 | 20-mesh wall | 3 | baglike, meshed | gypsum paste | slight leak |
| 3 | 20-mesh wall | 4 | baglike, meshed | gypsum paste | No leak |
| 4 | 50-mesh wall | 2 | baglike, meshed | PMMA | No leak |
| 5 | 50-mesh wall | 3 | baglike, meshed | PMMA | No leak |
| 6 | 100-mesh wall | 1 | baglike, non-woven fabric | PMMA | No leak |

The filling member of the present invention is filled with the pasty medicinal material by means of an injecting tool, such as an electric or manual injection cylinder. The pasty medicinal material is preferably capable of hardening. The recommended medicinal materials include bone cement, PMMA, and bone substitute such as gypsum and calcium phosphate.

The flexible and breathable filler of the present invention is used mainly for the medical blockage, the medical fixation, or the press of the soft tissues. The pasty medicinal material of the filler may be impregnated with a medical substance for treating disease or relieving pain. The medical substance is slowly released by the medicinal material.

The filling member of the present invention may be integrally formed of the multi-layer walls, or may be formed by connecting pieces of the multi-layer walls. The meshes of each layer of the multi-layer walls are not aligned. The filling member of the present invention is made of a biocompatible material, such as titanium, or a biodegradable material.

The features and the advantages of the present invention will be more readily understood upon a thoughtful deliberation of the following detailed description of the preferred embodiments of the present invention with reference to the accompanying drawings.

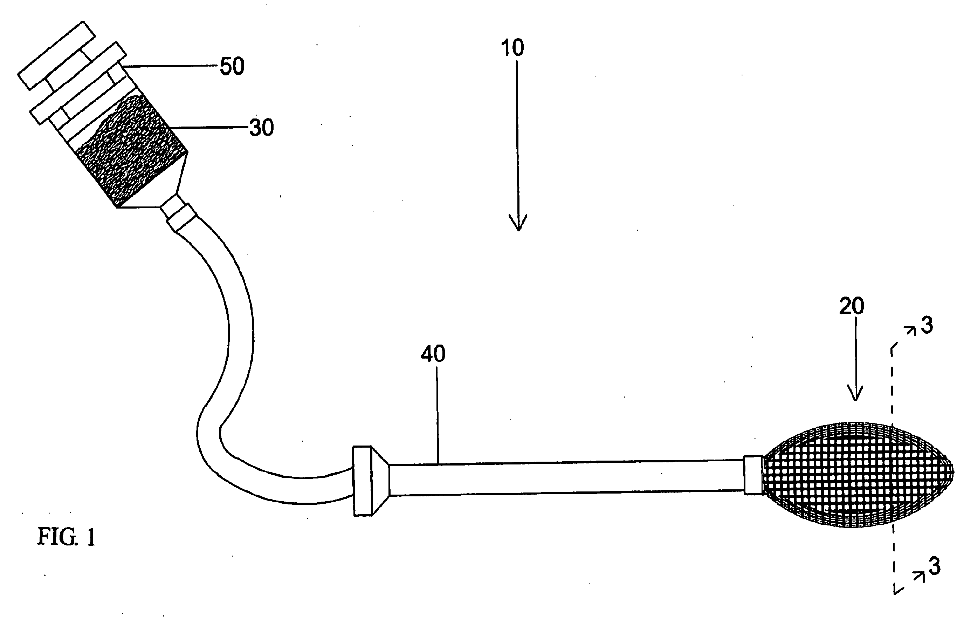

As shown in FIG. 1, a medical filler system 10 embodied in the present invention comprises a flexible and breathable filling member 20, a pasty medicinal material 30, a connection tube 40, and an injecting cylinder 50 by which the pasty medicinal material 30 is injected into the filling member 20 via the connection tube 40.

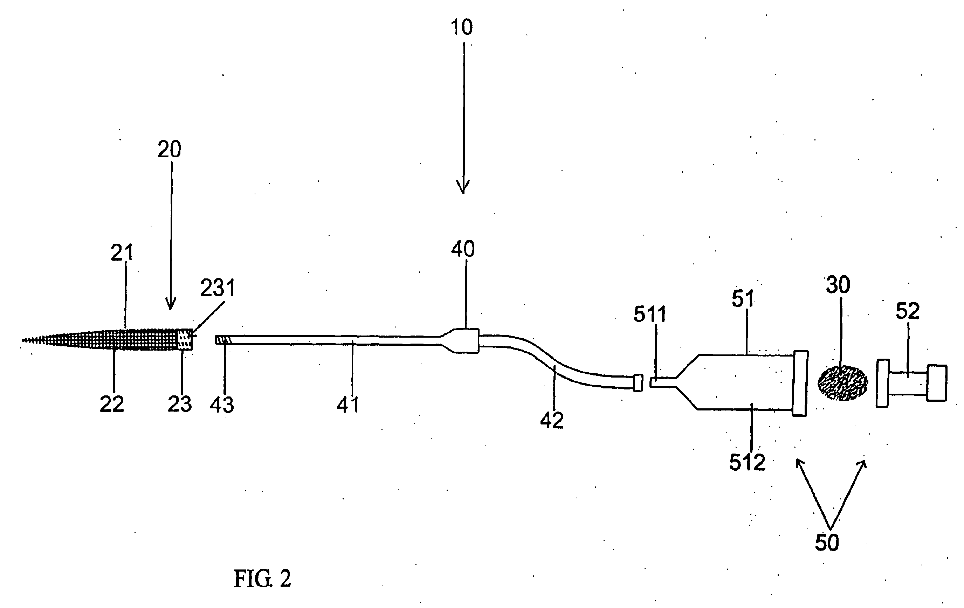

As shown in FIG. 2, the filling member 20 not forming part of the present invention is integrally made of a flexible and breathable single-layer wall 21 by punching and pressing and is provided with a hollow receiving portion 22 and an inlet 23 in communication with the receiving portion 22. The inlet 23 is provided with an inner threaded portion 231.

The pasty medicinal material 30 is preferable capable pf hardening. Prior to being injected into the filling member 20, the pasty medicinal material 30 is held in a barrel 51 of the injecting cylinder 50. The material 30 is injected by a plunging motion of a plunger 52 into the filling member 20 via the connection tube 40. The connection tube 40 is provided at one end 41 with an outer threaded portion 43 and is fastened at the one end 41 with the inlet 23 of the filling member 20 in such a manner that the outer threaded portion 43 of the connection tube 40 is engaged with the inner threaded portion 231 of the inlet 23. The connection tube 40 is fastened at another end 42 with an outlet 511 of the barrel 51.

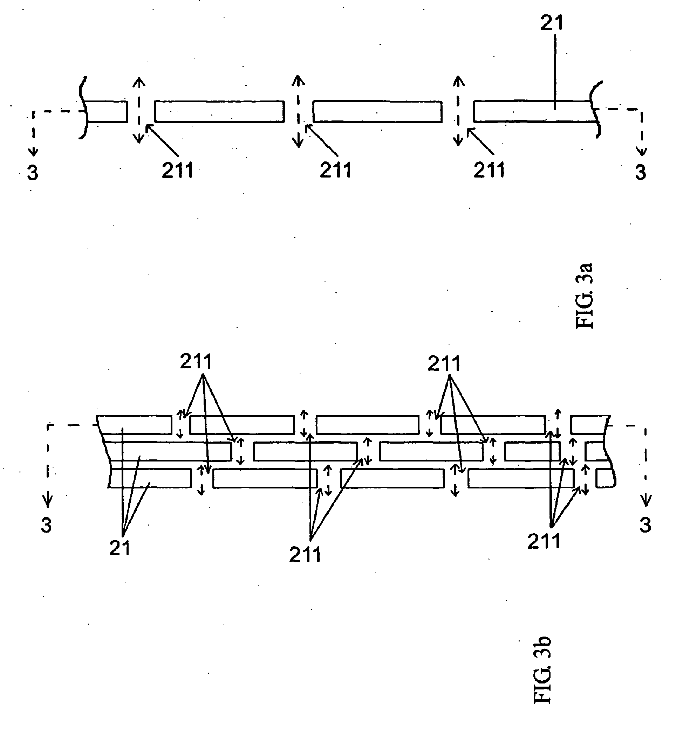

As shown in FIGS. 3a, the wall 21 of a filling member 20 not forming part of the present invention is formed of one layer. The wall 21 is provided with a plurality of meshes 211 pervious to gas or liquid and impermeable to solid. The filling member 20 is elastically compressible by virtue of the elasticity of the wall 21.

As shown in FIG. 3b, the wall 21 of the invention filling member are multi-layer laminated wall, wherein the meshes 211 of two adjacent layers are not aligned. As a result, the passage of gas or liquid through the walls 21 is slowed down by the nonalignment of the meshes 211. The meshes 211 are impermeable to the solid constituent of the material 30, thereby resulting in the walls 21 being pushed by the solid constituent.

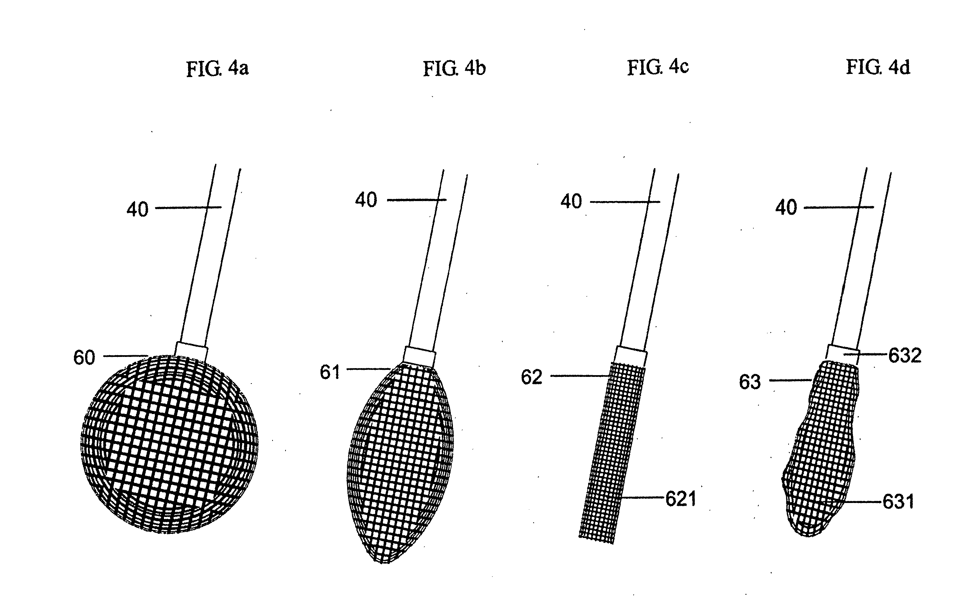

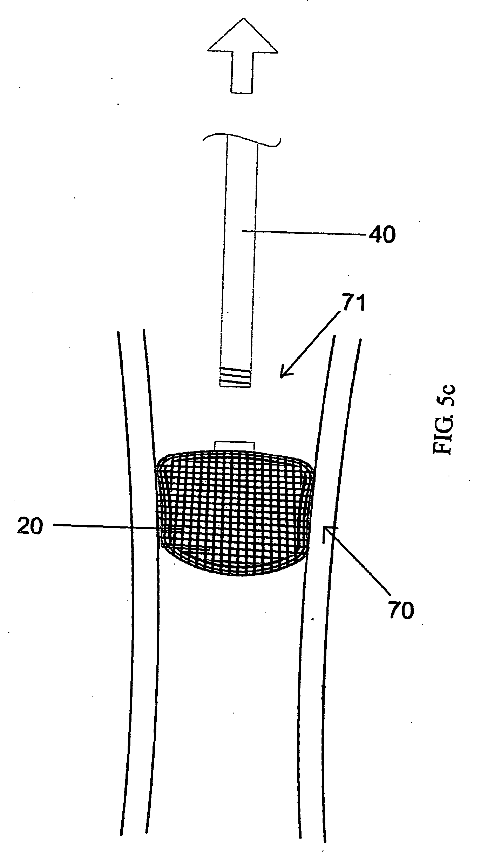

As illustrated in FIGS. 4a-4d, the filling member of the present invention takes various forms, such as spherical 60, oval 61, rod-shaped 62, and irregular-shaped 63. The rod-shaped member 62 is formed of a plurality of walls 621, which are so connected to form a rod shape. The irregularly-shaped member 63 is provided with a receiving portion 631 and an inlet 632.

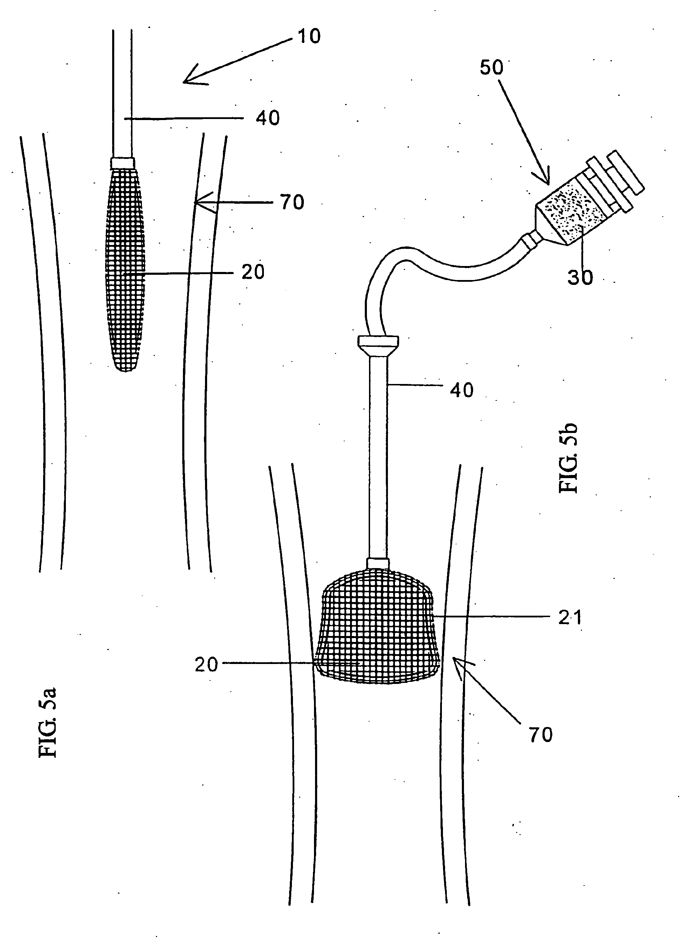

As illustrated in FIG. 5a, the filling member 20 is first compressed so as to enable it to be inserted into a bone cavity 70. Thereafter, the filling member 20 is filled with the pasty medicinal material 30, which causes the filling member 20 to regain its original form, as illustrated in FIG. 5b. As the pasty medicinal material 30 has hardened inside the filling member 20, the connection tube 40 is unfastened with the filling member 20, as illustrated in FIG. 5c. The bone cavity 70 is thus blocked by the filling member 20 so as to facilitate the planting of an artificial implant, such as an artificial hip joint, in an upper portion 71 of the bone cavity 70. The filling member 20 serves as a means to support the artificial hip joint.

## Claims

1. A medical filler comprising:
a flexible and breathable filling member (20) formed of laminated multi-layer walls (21) and provided with a hollow receiving portion (22) and an inlet (23) in communication with said receiving portion (22); and
a pasty medicinal material (30) to be injected into said receiving portion (22) via said inlet (23) of said filling member (20),
wherein breathable means pervious to gas or liquid and impermeable to the solid constituents of the pasty medicinal material (30),
said multi-layer walls (21) are elastically compressible and each layer is provided with a plurality of meshes (211), so that said multi-layer walls (21) are not air-tight,
**characterized in that**
the meshes (211) of two adjacent layers of a wall are not aligned.

2. The medical filler as defined in claim 1, wherein said each layer is provided with more than 6 meshes per 2.54 linear cm (linear inch).

3. The medical filler as defined in claim 1, wherein said each layer is provided with more than 20 meshes per 2.54 linear cm (linear inch).

4. The medical filler as defined in claim 1, wherein said each layer is provided with more than 40 meshes per 2.54 linear cm (linear inch).

5. The medical filler as defined in claim 1, wherein said each layer is provided with more than 100 meshes per 2.54 linear cm (linear inch).

6. The medical filler as defined in claim 1, wherein said filling member (20) is shaped like a sac, bag, ball(60), rod (62), or cylinder.

7. A medical filler system (10) comprising:
a medical filler according to claim 1; and
an injecting tool,
wherein said injecting tool is to be detachable fastened with said inlet (23) of said filling member (20), so that said pasty medicinal material (30) is able to be injected into said receiving portion (22) via said inlet (21) of said filling member (20) by said injecting tool.

8. The medical filler system as defined in claim 7, wherein said injecting tool comprises:
a connection tube (40) and
an injecting cylinder (50),
said connection tube (40) being provided at one end (41) with an outer threaded portion (43) such that said outer threaded portion (43) of said connection tube (40) is engaged with an inner threaded portion (231) of said inlet (23) of said filling member (20);
said injecting cylinder (50) comprising a barrel (51) and a plunger (52),
said barrel (51) being fastened with another end (42) of said connection tube (40),
wherein said barrel (51) is used to hold said pasty medicinal material (30) which is to be injected into said receiving portion (22) of said filling member (20) via said connection tube (40) by a plunging motion of said plunger (52).

9. The medical filler system as defined in claim 7, wherein said filling member (20) is shaped like a sac, bag, ball (60), rod (62), or cylinder.

## Patentansprüche

1. Medizinische Füllvorrichtung, die umfasst:
ein flexibles und atmungsaktives Füllelement (20), das aus laminierten mehrschichtigen Wänden (21) besteht und mit einem hohlen Aufnahmeabschnitt (22) sowie einem Einlass (23) versehen ist, der in Verbindung mit dem Aufnahmeabschnitt (22) steht; und
ein pastöses medizinisches Material (30), das über den Einlass (23) des Füllelementes (20) in den Aufnahmeabschnitt (22) einzuspritzen ist,
wobei die atmungsaktive Einrichtung für Gas oder Flüssigkeit durchlässig und für feste Bestandteile des pastösen medizinischen Materials (30) undurchlässig ist,
die mehrschichtigen Wände (21) elastisch zusammengedrückt werden können und jede Schicht mit einer Vielzahl von Maschen (211) versehen ist, so dass die mehrschichtigen Wände (21) nicht luftdicht sind,
**dadurch gekennzeichnet, dass**
die Maschen (211) zweier aneinander grenzender Schichten einer Wand nicht fluchtend sind.

2. Medizinische Füllvorrichtung nach Anspruch 1, wobei jede Schicht mit mehr als 6 Maschen pro 2,54 lineare Zentimeter (linearer Inch) versehen ist.

3. Medizinische Füllvorrichtung nach Anspruch 1, wobei jede Schicht mit mehr als 20 Maschen pro 2,54 lineare Zentimeter (linearer Inch) versehen ist.

4. Medizinische Füllvorrichtung nach Anspruch 1, wobei jede Schicht mit mehr als 40 Maschen pro 2,54 lineare Zentimeter (linearer Inch) versehen ist.

5. Medizinische Füllvorrichtung nach Anspruch 1, wobei jede Schicht mit mehr als 100 Maschen pro 2,54 lineare Zentimeter (linearer Inch) versehen ist.

6. Medizinische Füllvorrichtung nach Anspruch 1, wobei das Füllelement (20) wie ein Sack, ein Beutel, eine Kugel (60), eine Stange (62) oder ein Zylinder geformt ist.

7. Medizinisches Füllsystem (10) das umfasst:
eine medizinische Füllvorrichtung nach Anspruch 1; und
ein Einspritzwerkzeug,
wobei das Einspritzwerkzeug abnehmbar an dem Einlass (23) des Füllelementes (20) anzubringen ist, so dass das pastöse medizinische Material (30) durch das Einspritzwerkzeug über den Einlass (21) des Füllelementes (20) in den Aufnahmeabschnitt (22) eingespritzt werden kann.

8. Medizinisches Füllsystem nach Anspruch 7, wobei das Einspritzwerkzeug umfasst:
eine Verbindungsröhre (40) und
einen Einspritzzylinder (50),
wobei die Verbindungsröhre (40) an einem Ende (41) so mit einem Außengewindeabschnitt (43) versehen ist, dass der Außengewindeabschnitt (43) der Verbindungsröhre (40) mit einem Innengewindeabschnitt (231) des Einlasses (23) des Füllelementes (20) in Eingriff gebracht wird;
der Einspritzzylinder eine Hülse (51) und einen Kolben (52) umfasst,
die Hülse (51) an einem anderen Ende (42) der Verbindungsröhre (40) befestigt ist,
und die Hülse (51) dazu dient, das pastöse medizinische Material (30) aufzunehmen, das durch eine Eindrückbewegung des Kolbens (52) über die Verbindungsröhre (40) in den Aufnahmeabschnitt (22) des Füllelementes (20) einzuspritzen ist.

9. Medizinisches Füllsystem nach Anspruch 7, wobei das Füllelement (20) wie ein Sack, ein Beutel, eine Kugel (60), eine Stange (62) oder ein Zylinder geformt ist.

## Revendications

1. Matière de remplissage médicale comprenant :
un élément de remplissage souple et respirant (20)' formé de parois multicouches stratifiées (21) et muni d'une partie de réception creuse (22) et d'une entrée (23) en communication avec ladite partie de réception (22) ; et
une substance médicamenteuse pâteuse (30) destiné à être injectée dans ladite partie de réception (22) par l'intermédiaire de ladite entrée (23) dudit élément de remplissage (20),
le mot "respirant" signifiant perméable au gaz ou aux liquides et imperméable aux constituants solides de la substance médicamenteuse pâteuse (30),
lesdites parois multicouches (21) sont compressibles élastiquement et chaque couche comporte plusieurs mailles (211) afin que lesdites parois multicouches (21) ne soient pas étanches à l'air,
**caractérisée en ce que**
les mailles (211) de deux couches adjacentes d'une paroi ne sont pas alignées.

2. Matière de remplissage médicale selon la revendication 1, dans laquelle chaque couche comporte plus de 6 mailles par 2,54 centimètres linéaires (pouce linéaire).

3. Matière de remplissage médicale selon la revendication 1, dans laquelle chaque couche comporte plus de 20 mailles par 2,54 centimètres linéaires (pouce linéaire).

4. Matière de remplissage médicale selon la revendication 1, dans laquelle chaque couche comporte plus de 40 mailles par 2,54 centimètres linéaires (pouce linéaire).

5. Matière de remplissage médicale selon la revendication 1, dans laquelle chaque couche comporte plus de 100 mailles par 2,54 centimètres linéaires (pouce linéaire).

6. Matière de remplissage médicale selon la revendication 1, dans laquelle ledit élément de remplissage (20) a la forme d' un sac, d'une poche, d'un ballon (60), d'une tige (62) ou d'un cylindre.

7. Système de remplissage médical (10) comprenant :
une matière remplissage médicale selon la revendication 1 ; et
un outil d'injection,
ledit outil d'injection étant destiné à être fixé de manière amovible à ladite entrée (23) dudit élément de remplissage (20) afin que ladite substance médicamenteuse pâteuse (30) puisse être injectée dans ladite partie de réception (22) par l'intermédiaire de ladite entrée (21) dudit élément de remplissage (20) par ledit outil d'injection.

8. Système de remplissage médical selon la revendication 7, dans lequel ledit outil d'injection comprend :
un tube de raccordement (40) et
un cylindre d'injection (50),
ledit tube de raccordement (40) étant muni au niveau de l'une de ses extrémités (41) d'une partie filetée extérieure (43) afin que celle-ci puisse être accouplée avec une partie filetée intérieure (231) de ladite entrée (23) dudit élément de remplissage (20) ;
ledit cylindre d'injection (50) comprenant un corps (51) et un piston (52),
ledit corps (51) étant fixé à l'autre extrémité (42) dudit tube de raccordement (40),
dans lequel ledit corps (51) est utilisé pour maintenir ladite substance médicamenteuse pâteuse (30) destinée à être injectée dans ladite partie de réception (22) dudit élément de remplissage (20) par l'intermédiaire dudit tube de raccordement (40) grâce à un mouvement de poussée dudit piston (52).

9. Système de remplissage médical selon la revendication 7, dans lequel ledit élément de remplissage (20) a la forme d'un sac, d'une poche, d'un ballon (60), d'une tige (62) ou d'un cylindre.
